# EUROPÄISCHE PATENTSCHRIFT

(11) **EP 3 026 048 B1**
(45) Veröffentlichungstag und Bekanntmachung des Hinweises auf die Patenterteilung: **25.07.2018**
(21) Anmeldenummer: 14195235.8
(22) Anmeldetag: 27.11.2014
(51) Int. Cl.: C07D 311/74, C07D 307/79, C11B 9/00

(54) **Hexahydrobenzofurane und Hexahydrochromene - Verfahren zur Herstellung und ihre Verwendung als Duftstoffe**
Hexahydrobenzofurans and hexahydrochromenes - method for production and their use as fragrance compounds
Hexahydrobenzofurane et hexahydrochromene - Procédé de fabrication et leur utilisation en tant que parfum

(43) Veröffentlichungstag der Anmeldung: 01.06.2016
(73) Patentinhaber: Symrise AG, 37603 Holzminden (DE)
(72) Erfinder: Hölscher, Bernd, 37620 Halle (DE); Panten, Johannes, 37671 Höxter (DE); Surburg, Horst, 37603 Holzminden (DE)
(74) Vertreter: Global IP Europe Patentanwaltskanzlei

(56) Entgegenhaltungen:
- JOHANNES PANTEN ET AL: "Recent Results in the Search for New Molecules with Ambergris Odor", CHEMISTRY & BIODIVERSITY, Bd. 11, Nr. 10, 20. Oktober 2014 (2014-10-20), Seiten 1639-1650, XP055170604, ISSN: 1612-1872, DOI: 10.1002/cbdv.201400153

## Beschreibung

### Gebiet der Erfindung

Die vorliegende Erfindung betrifft neue Duftstoffe, Verfahren zu ihrer Herstellung, Parfüms und Konsumgüter enthaltend diese Duftstoffe sowie deren Verwendungen.

### Stand der Technik

In der Parfümindustrie sind eine Vielzahl verschiedenster Verbindungen bekannt, die zur Vermittlung eines Wohlgeruchs eingesetzt werden. Es besteht der stetige Bedarf nach neuartigen vorteilhaften Verbindungen, die neben ihren positiven und originellen geruchlichen Eigenschaften zusätzliche positive Sekundäreigenschaften besitzen.

Bei der Suche nach geeigneten Verbindungen wird der Fachmann vor die schwierige Aufgabe gestellt, aus einer nahezu unendlich erscheinenden Anzahl möglicher Verbindungen, geeignete Duftstoffe auszuwählen. Dabei lässt sich nicht vorhersagen, ob unbekannte Verbindungen überhaupt einen Geruch aufweisen und ob dieser Geruch erwünschte oder unerwünschte geruchliche Eigenschaften aufweist. Beim Auffinden einer Verbindung mit positivem Geruch ist zudem sehr fraglich, um welchen Geruch es sich handelt und inwieweit die Verbindung auch über positive sekundäre Eigenschaften verfügt. Viele bisher bekannte Duftstoffe weisen zum Teil deutliche Nachteile auf, beispielsweise besitzen diese eine sehr beschränkte Stabilität und Ausgiebigkeit, ein geringes Haftungsvermögen, eine geringe Ausstrahlungskraft, eine schlechte Löslichkeit und müssen in hohen Dosierungen eingesetzt werden.

An neue Duftstoffe werden sehr hohe Anforderungen gestellt. Sie sollten eine sehr gute biologische Abbaubarkeit aufweisen und dermatologisch sowie toxikologisch unbedenklich sein.

Der Geruch "ambra" oder auch "amber" stellt eine der hochbevorzugten Geruchsnoten in Parfüms dar. Er verleiht Parfüms eine besondere Exklusivität und Luxuriosität. Ursprünglich wurde Amber aus dem Verdauungstrakt von Pottwalen gewonnen, heutzutage werden Ambra-Düfte aus anderen Quellen bevorzugt. Nachteilig an vielen Ambra-Duftstoffen, ist, dass ihr Geruchseindruck erst allmählich wahrgenommen wird, weshalb es schwer ist, den Ambrageruch in der Topnote eines Parfüms darzustellen.

Holzige Geruchsnoten werden sehr oft in Parfüms verwendet und zählen zu den sehr beliebten Geruchsnoten. Ambranoten, die zudem auch holzig riechen werden als besonders hochwertig empfunden.

### Aufgabe der Erfindung

Die Aufgabe der Erfindung bestand darin, neue Verbindungen zur Verfügung zu stellen, welche die eingangs genannten Nachteile des Standes der Technik nicht aufweisen oder abmildern. Insbesondere bestand die Aufgabe der Erfindung darin, Duftstoffe zur Verfügung zu stellen, welche die Geruchsnote "ambra" und / oder holzig darstellen können und darüber hinaus über positive sekundäre Eigenschaften verfügen.

### Lösung der Aufgabe

Die Aufgabe wird gelöst durch Verbindungen der Formel I: 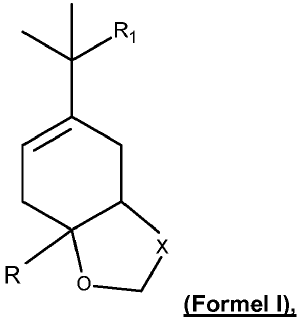 wobei die variablen Reste die folgende Bedeutung haben:
R = -methyl oder -ethyl
R₁₌-H, -methyl, -ethyl, O-methyl oder O-ethyl und
X= -CH2- oder -CH2-CH2-.

Die erfindungsgemäßen Stoffe können als 5,7a-Dialkyl-hexahydro-benzofurane bzw. 6,8a-Dialkyl-hexahydro-chromene bezeichnet werden.

Überraschend weisen die erfindungsgemäßen Verbindungen eine ambra-und/oder holzige Geruchsnote auf. Darüber hinaus weisen sie eine hohe Stabilität und Ausgiebigkeit, ein sehr gutes Haftungsvermögen, eine sehr große Ausstrahlungskraft, einen niedrigen Geruchsschwellenwert, eine sehr gute Löslichkeit und Mischbarkeit, eine geringe Neigung zu Reaktionen mit anderen Duftstoffen, eine sehr gute dermatologische und toxikologische Verträglichkeit sowie eine sehr gute biologische Abbaubarkeit auf.
Ein weiterer Vorteil der erfindungsgemäßen Verbindungen ist ihre hohe Duftintensität bei vergleichsweise geringer Dosierung. Dies ist aus Gründen der Umweltreinhaltung von besonderem Interesse, da hierdurch die Menge der in die Umwelt freigesetzten Stoffe minimal gehalten werden kann. Weiterhin wird die Ambranote besonders schnell wahrgenommen, so dass sich die Verbindungen beispielsweise als Topnote in einem Parfümöl eignen.

Die Verbindungen der Formel I selbst sind sehr stabil und weisen ebenso eine hohe Stabilität unter ungünstigen Bedingungen, wie hohem Sauerstoffgehalt, hohem Gehalt an Oxidationsmitteln oder Reduktionsmitteln, hohen Temperaturen und unter extremen pH-Bedingungen auf.

Die erfindungsgemäßen Verbindungen können als Racemate oder einzelne Enantiomere vorliegen.

Bevorzugt beträgt das Molgewicht der erfindungsgemäßen Verbindungen mindestens 180 und höchstens 238 g/mol.

Weiter bevorzugte Verbindungen sind Verbindungen der Formel I, wobei die variablen Reste die folgende Bedeutung haben:
R = -methyl oder -ethyl,
R₁₌-H, -methyl, -ethyl, O-methyl oder O-ethyl und
X= -CH2- oder -CH2-CH2-.

Insbesondere bevorzugt sind diese konkreten Verbindungen:

**Tabelle 1: Konkrete erfindungsgemäße Verbindungen sowie deren Geruchsbeschreibungen**

| **Verbindung** | **Molgewicht** | **Struktur** | **Geruch** |
|---|---|---|---|
| **1** | **180** | 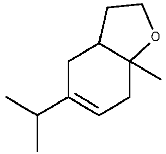 | ambra, holzig |
| **2** | **194** | 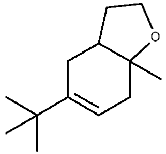 | ambra, holzig |
| **3** | **210** | 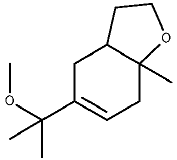 | ambra, holzig |
| **4** | **208** | 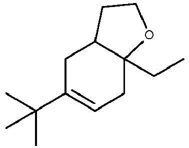 | ambra, holzig |
| **5** | **208** | 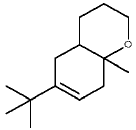 | ambra, holzig |
| **6** | **194** | 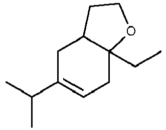 | ambra, holzig |
| **7** | **208** | 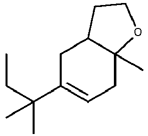 | ambra, holzig |
| **8** | **194** | 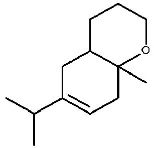 | ambra, holzig |

Die Verbindungen zeichnen sich dadurch aus, dass sie bei geringer Dosierung einen besonders komplexen, strahlenden und hochintensiven Ambraduft aufweisen. Darüber hinaus weisen sie einen holzigen Duft auf, so dass die besonders erwünschte Kombination aus Ambra- und Holznoten entsteht. Die Erfindung betrifft die Verbindungen als solche, einzeln oder auch Mischungen der erfindungsgemäßen Verbindungen.

Ein weiterer Gegenstand der Erfindung betrifft Mischungen enthaltend oder bestehend aus einer oder mehreren erfindungsgemäßen Verbindungen (Komponente a) und einem oder mehreren Lösungsmitteln (Komponente b) sowie optional einem oder mehreren Duftstoffen (Komponente c). Die erfindungsgemäßen Verbindungen sind gut in den verschiedensten Lösungsmitteln löslich. Bevorzugte Lösungsmittel, welche die erfindungsgemäßen Verbindungen besonders gut zu lösen vermögen sind ausgewählt aus der Gruppe, die gebildet wird von Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat sowie deren Mischungen. Der Anteil der erfindungsgemäßen Verbindungen (Komponenten a) in diesen Mischungen beträgt bevorzugt 0,1 bis 50 Gew.-%, weiter bevorzugt 0,5-15 Gew.-% und besonders bevorzugt 0,75 bis 2 Gew.-%, bezogen auf das Gesamtgewicht der erfindungsgemäßen Verbindungen (Komponenten a) und der Lösungsmittel (Komponenten b). Gemische aus den Komponenten a, b und c bleiben auch bei sehr kalten Temperaturen homogen. Dies ist wichtig, um etwa Transporte im Winter schadlos zu überstehen.

Außerdem betrifft die Erfindung Mischungen enthaltend oder bestehend aus den erfindungsgemäßen Verbindungen (Komponenten a) und einem oder mehreren Duftstoff(en) (Komponenten c). Die erfindungsgemäßen Verbindungen sind besonders gut und in einem sehr weiten Konzentrationsverhältnis in anderen Duftstoffen löslich und weisen eine geringe Reaktivität mit anderen Duftstoffen auf. Die erfindungsgemäßen Verbindungen wirken in diesen Mischungen abrundend, harmonisierend und verbessern den natürlichen Geruchseindruck. Das Verhältnis aus der Gesamtmasse der erfindungsgemäßen Verbindungen (Komponenten a) zur Gesamtmasse der anderen Duftstoffe (Komponenten c) beträgt bevorzugt von 1:999 bis 999:1, weiter bevorzugt von 1:99 bis 1:1, insbesondere bevorzugt von 1:99 bis 1:2, weiter insbesondere bevorzugt 1:90 bis 1:5 und besonders bevorzugt von 1:80 bis 1:10.

Beispiele für Duftstoffe, mit denen die erfindungsgemäßen Verbindungen vorteilhaft kombiniert werden können, finden sich z. B. in "S. Arctander, Perfume and Flavor Chemicals, Vol. I und II, Montclair, N. J., 1969, Selbstverlag" oder "H. Surburg und J. Panten, Common Fragrance and Flavor Materials, 5th ed., Wiley-VCH, Weinheim, 2006". Im Einzelnen seien genannt:
**Extrakte aus natürlichen Rohstoffen** wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreumabsolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora-ÖI; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
**Einzel-Riechstoffe** aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die Erfindung betrifft ebenso Mischungen, die erfindungsgemäße Verbindungen (Komponenten a), ein oder mehrere Lösungsmittel (Komponenten b) sowie optional einen oder mehrere Duftstoffe (Komponenten c) enthalten oder aus diesen bestehen. Insbesondere handelt es sich bei solchen Mischungen um Parfümöle, Parfüms, Parfüm-Extraits, Eau de Parfums, Eau de Toilettes, Rasierwässer, Eau de Colognes, Pre-shave-Produkte, Splash-Colognes. Die erfindungsgemäßen Verbindungen bilden dabei insbesondere die Topnote der Parfüms oder der Parfümöle. Zur Topnote eines Parfümöls gehören diejenigen Noten, deren Geruch besonders schnell wahrgenommen werden kann. Bezogen auf die Gesamtmasse der Komponenten a, b und c beträgt der Massenanteil der Komponenten a und c zusammen bevorzugt 0,1 bis 50 Gew.-%, wobei das Massenverhältnis der Komponenten a zu c im Bereich 1:90 bis 1:5 liegt.

Ferner betrifft die Erfindung Konsumgüter einschließlich deren Vor- und Zwischenprodukte enthaltend die erfindungsgemäßen Verbindungen oder deren Mischungen mit Lösungsmitteln oder/und weiteren Duftstoffen. Konsumgüter enthalten oft eine Vielzahl unterschiedlicher chemischer Verbindungen. Der Einsatz der erfindungsgemäßen Verbindungen ist insbesondere vorteilhaft wegen deren geringer Reaktivität, so dass weder die erfindungsgemäßen Verbindungen noch die Inhaltsstoffe der Konsumgüter nachteilig verändert werden. Zudem beeinflussen die erfindungsgemäßen Verbindungen in den zur Erzielung der erwünschten Duftwirkung notwendigen Konzentrationen nicht nachteilig die physikalischen oder physikochemischen Eigenschaften der Konsumgüter (z.B. die Viskosität oder den pH-Wert). Der Massenanteil der erfindungsgemäßen Verbindungen in den Konsumgütern beträgt bevorzugt 0,0001 bis 5 Gew.-%, weiter bevorzugt 0,001 bis 2 Gew.-% und besonders bevorzugt 0,001 bis 1 %.

Konsumgüter im Sinne der Erfindung sind insbesondere Kosmetika, Wasch- und Reinigungsmittel, Weichspüler, Bleichmittel, Desinfektionsmittel, Duftfreigabesysteme, Sonnenschutzmittel für die Haut, Haarpflegemittel und Deodorants. Besonders handelt es sich bei Konsumgütern um saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel sowie Luftverbesserer in flüssiger, gelartiger oder auf einem festen Träger aufgebrachter Form einschließlich zur Beduftung von Toiletten, Aerosolsprays, Wachse und Polituren wie Möbelpolituren, Fußbodenwachse, Schuhcremes sowie Körperpflegemittel wie z.B. feste und flüssige Seifen, Duschgele, Shampoos, Rasierseifen, Rasierschäume, Badeöle, kosmetische Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-Shaves, After Shave-Cremes und Lotionen, Pre-Shaves, Bräunungscremes und -lotionen, Haarpflegeprodukte wie z.B. Haarsprays, Haargele, Haarlotionen, Haarspülungen, permanente und semipermanente Haarfärbemittel, Haarverformungsmittel wie Kaltwellen und Haarglättungsmittel, Haarwässer, Haarcremes und -lotionen, Erfrischungstücher, Deodorantien und Antiperspirantien wie z.B. Achselsprays, Roll-ons, Deosticks, Deocremes oder Produkte der dekorativen Kosmetik wie zum Beispiel Make-Up.

Bei den in Konsumgütern enthaltenen Stoffen handelt es sich oftmals um Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate. Mit diesen Stoffen weisen die erfindungsgemäßen Verbindungen in Mischungen eine hohe Kompatibilität auf.

Die Erfindung betrifft auch die Verwendung der erfindungsgemäßen Stoffe. Die erfindungsgemäßen Stoffe können als Duftstoffe verwendet werden, insbesondere zur Erzeugung, zum Vermitteln, Modifizieren oder Verstärken eines ambrartigen und / oder holzigen Geruches. Besonders bevorzugt können die erfindungsgemäßen Verbindungen verwendet werden zur Erzeugung, zum Vermitteln, Modifizieren oder Verstärken einer ambrartigen und / oder holzigen Topnote eines Parfümöls. Außerdem lassen sich die erfindungsgemäßen Verbindungen zur Abrundung, Harmonisierung und zur Verbesserung der Natürlichkeit von Parfümölen und diesen enthaltenden Konsumprodukten einsetzen.

Neben dem Einsatz als Flüssigkeit in Lösungen oder in Emulsionen können die erfindungsgemäßen Verbindungen oder diese enthaltende Parfümöle an Feststoffen adsorbiert oder in Trägerstoffen (mikro)verkapselt eingesetzt werden. Diese Darreichungsformen können sowohl für eine feine Verteilung der Riechstoffe im Produkt als auch für eine kontrollierte Freisetzung bei der Anwendung sorgen. Derartige Feststoffe können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein. Verkapselungsprodukte können beispielsweise sprühgetrocknet, als Einschluß-Komplex oder als Extrusions-Produkt vorliegen. Die Mikroverkapselung der Parfümöle kann beispielsweise durch das sogenannte Koazervationsverfahren mit Hilfe von Kapselmaterialien z.B. aus polyurethanartigen Stoffen oder Weichgelatine erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Die Adsorbate als auch die Verkapselungsprodukte können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden können.

Die Erfindung betrifft auch Verfahren zur Herstellung der erfindungsgemäßen Verbindungen, wobei mehrere alternative Syntheserouten möglich sind. Ausgehend von Verbindungen der Formel II 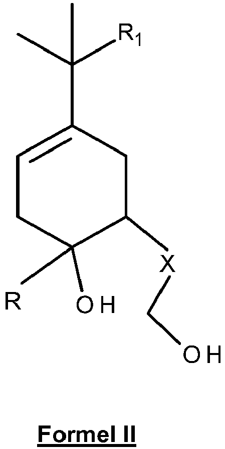 worin die variablen Reste
R = -methyl oder -ethyl
R₁ = -H, -methyl, -ethyl, O-methyl, O-ethyl
X= -CH2- oder -CH2-CH2-
bedeuten, erhält man die erfindungsgemäßen Verbindungen durch intramolekulare Cyclisierung. R, R1 und X werden dabei analog zur herzustellenden erfindungsgemäßen Verbindung gewählt.

Die Verbindungen der Formel II sind entweder kommerziell erhältlich oder können durch Synthese gewonnen werden. Es sind mehrere Syntheserouten zur Herstellung der Verbindungen der Formel II möglich:

### Syntheseroute Ia:

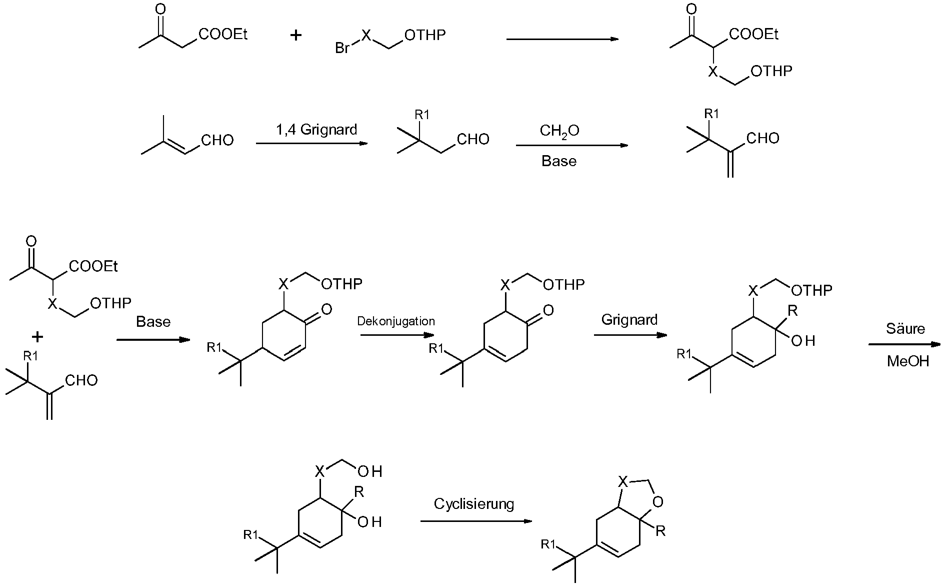

### Syntheseroute Ib:

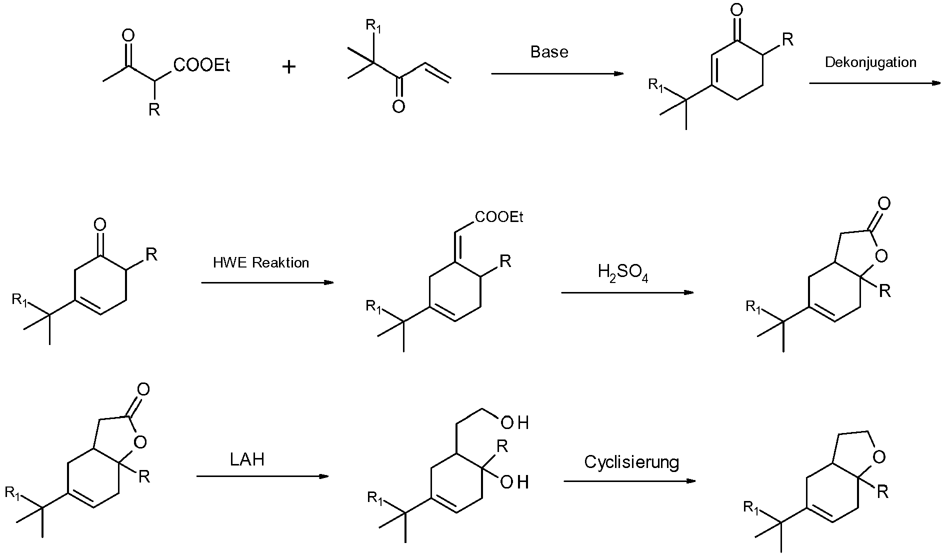

Die Verbindungen der Formel I lassen sich alternativ durch Dehydratisierung von Verbindungen der Formel III Herstellen:

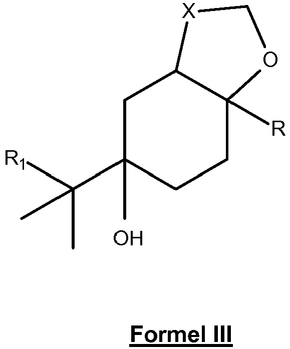

R, R1 und X werden dabei analog zur herzustellenden erfindungsgemäßen Verbindung gewählt. Die Verbindungen der Formel III sind entweder kommerziell erhältlich oder können durch die Syntheseroute II hergestellt werden.

### Syntheseroute II:

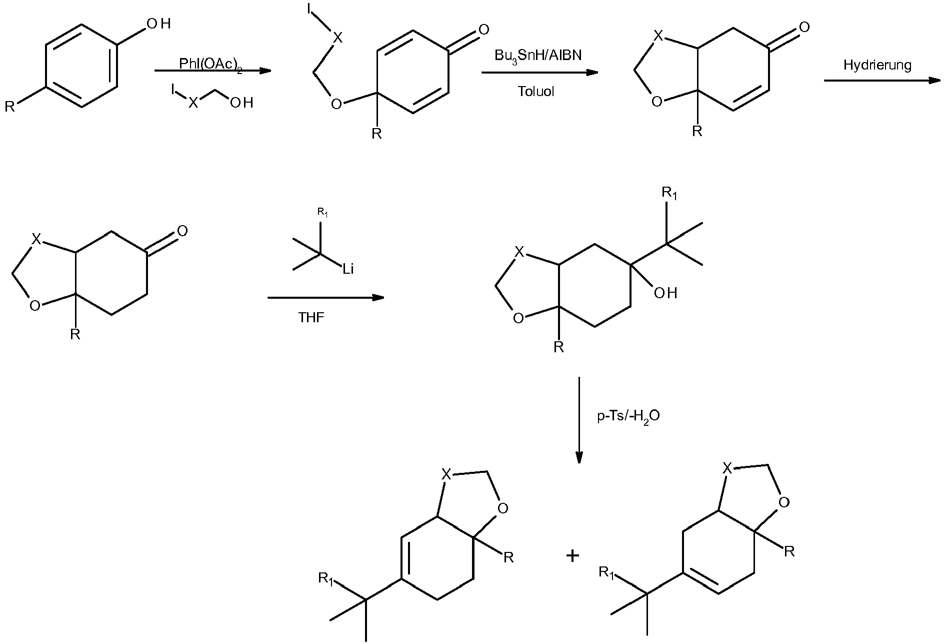

Es wurde festgestellt, dass die intramolekulare Cyclisierung von Verbindungen der Formel II oder/und die Dehydratisierung von Verbindungen der Formel III zu Verbindungen der Formel I besonders gut, d.h. insbesondere mit hoher Ausbeute und in einem kurzen Zeitraum, in Gegenwart von Sulfonsäure oder deren Derivaten abläuft. Bevorzugt sind Sulfonsäure und Sufonsäurehalogenide und insbesondere bevorzugt Sulfonsäure, Sulfonsäurechlorid, Toluolsulfonsäurechlorid, Benzolsulfonsäurechlorid sowie p- Toluolsulfonsäurechlorid. Weiterhin wirken sich die Gegenwart von Tris(dioxa-3,6-heptyl)amin oder/und die Verwendung von Toluol als Lösungsmittel positiv auf die Reaktionen aus. Bevorzugte Temperaturen sind 30°C bis maximal 65°C und besonders bevorzugt 50-60°C.

Die Erfindung betrifft auch ein Verfahren zur Parfümierung von Konsumgütern umfassend das Hinzufügen einer erfindungsgemäßen Verbindung oder den erfindungsgemäßen Mischungen zu Konsumgütern. Der Vorteil der erfindungsgemäßen Verbindungen oder der erfindungsgemäßen Mischungen bei diesem Verfahren ist, dass sie sich leicht in Konsumgüter einarbeiten lassen.

### Beispiel 1

Herstellung von 5-tert-butyl-7a-methyl-3,3a,4,7-tetrahydro-2H-benzofuran (Verbindung 2) über die Syntheseroute la:

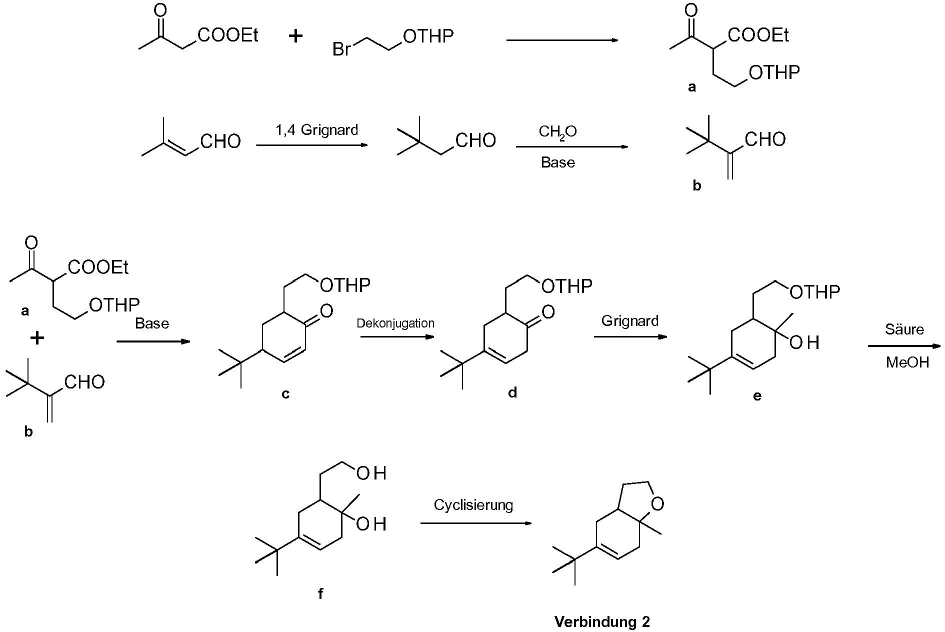

### Synthese von Zwischenverbindung a

136 g (2mol) Natriumethylat in 800 g Ethanol abs. werden unter Rühren und Kühlen bei Raumtemperatur mit 260 g (2mol) Acetessigsäureethylester versetzt und 1h nachgerührt. Anschließend tropft man bei 0°C innerhalb von 5h unter Rühren und Kühlen 418 g (2mol) 2-(2-Bromethoxy)Tetrahydropyran zu. Man rührt noch 3h bei Raumtemperatur und 4 h unter Rückfluss nach. Danach filtriert man das ausgefallene NaBr ab und engt die Reaktionslösung ein. Der Rückstand wird in 800ml Methyl-tert.-Butylether aufgenommen und mit Wasser neutral gewaschen und eingeengt. Die Rohware wird an einer kleinen Kolonne destilliert. Ausbeute : 336,9 g (65,3% d. Th.) an Zwischenverbindung a.

### Synthese von Zwischenverbindung b

Zu einer gekühlten Suspension aus 23,2g Cu(I)J in 200ml THF abs, werden bei -5°C (2mol) Methylmagnesiumbromid in THF abs. getropft dann wird noch 1h bei gleicher Temperatur nachgerührt. Anschließend werden bei -20°C 168g (1mol) 3-Methylbut-2-enal in 200ml THF abs. zu getropft. Über Nacht wird bei gleicher Temperatur nachgerührt. Die Reaktionsmischung wird in 1l eiskalte, gesättigte Ammonium-chlorid-Lsg. gegeben und nach Abtrennung der wässrigen Phase noch 2mal mit je 150ml gesättigter Kochsalz-Lsg. neutral gewaschen. Nach Abdestillieren des Lösungsmittels wird der Rückstand an einer kleinen Kolonne destilliert. Ausbeute: 141,2g (70,6% d. Th.) 3,3-Dimethylbutanal.

Zu 110g (1,1mol) Formaldehyd 30%ig und 3,5g Di-n-Butylamin wird bei 90°C 100g (1mol) 3,3-Dimethylbutanal getropft. Anschließend 3h bei gleicher Temperatur nachrühren. Die Abgekühlte Reaktionsmischung wird in 100ml Cyclohexan aufgenommen und 2mal mit je 75ml 5%iger Schwefelsäure bzw. 2mal mit je 75ml 5%iger Soda-Lsg. gewaschen. Nach Abdestillieren des Lösungsmittels wird der Rückstand an einer kleinen Kolonne destilliert. Ausbeute: 103,4g (92,3% d. Th.) an Zwischenverbindung b.

### Synthese von Zwischenverbindung c

Zu 88g (1,1mol) wässrigen NaOH 50%igen in 900ml Ethanol wird bei 0°C 258g (1mol) Zwischenverbindung a und anschließend 112g (1mol) Zwischenverbindung b getropft. Das Reaktionsgemisch wird 1 Stunde am Rückfluss erhitzt und anschließend das Lösungsmittel abdestilliert. Der Rückstand wird mit Wasser (300 ml) und MtBE (300 ml) versetzt und mit 20%iger wässriger Schwefelsäure neutralisiert die Phasen werden getrennt. Die organische Phase wird mit Soda-Lsg. neutral gewaschen und eingeengt. Die Rohware wird an einer kleinen Kolonne destilliert. Ausbeute : 154,3g (55,1% d. Th.) an Zwischenverbindung c.

### Synthese von Zwischenverbindung d

140g (1mol) Zwischenverbindung c wird bei 160°C mit 10mol% Adipinsäure 5h gerührt. Das Reaktionsgemisch wird mit 300ml Wasser und 300ml MtBE versetzt. Die Phasen werden getrennt, die organische Phase wird mit Soda-Lsg. neutral gewaschen und eingeengt. Die Rohware wird an einer kleinen Kolonne destilliert. Ausbeute : 106,1g (75,8% d. Th.) an Zwischenverbindung d.

### Synthese von Zwischenverbindung e

Zu 0,3mol Methylmagnesiumbromid in THF abs. wird bei 0°C eine Lösung aus 70g (0,25mol) Zwischenverbindung d in 200ml THF abs. getropft. Anschließend 3h bei Raumtemperatur nachrühren. Die Reaktionsmischung wird in 250ml eiskalte, gesättigte Ammoniumchlorid-Lsg. gegeben und nach Abtrennung der wässrigen Phase noch 2mal mit je 50ml gesättigter Kochsalz-Lsg. neutral gewaschen. Nach Abdestillieren des Lösungsmittels wird der Rückstand an einer kleinen Kolonne destilliert. Ausbeute : 54,6g (73,8% d. Th.) an Zwischenverbindung e.

### Synthese von Zwischenverbindung f

29,6g (0,1mol) Zwischenverbindung e in 200ml Methanol und 20g Molekularsieb wird über Nacht bei Raumtemperatur gerührt. Das Molekularsieb wird abfiltriert und mit Soda-Lsg. neutral gewaschen und eingeengt. Der Rückstand wird an einer kleinen Kolonne destilliert. Ausbeute : 17,2g (81,1% d. Th.) an Zwischenverbindung f.

### Synthese von Verbindung 2

10,6g (0,05mol) Zwischenverbindung f in 100ml Toluol und 10g NaOH 50%ig wird auf 60°C erwärmt. Anschließend wird unter Rühren bei 60°C 13g (0,7mol) Tosylchlorid in 30ml Toluol zu getropft und 4h bei gleicher Temperatur nachgerührt. Die Reaktionslösung wird mit 50ml Wasser versetzt und 2h unter Rückfluss gerührt. Danach die wässrige Phase abziehen, die organische Phase 2mal mit je 50ml Wasser waschen und die Toluolphase einengen. Der Rückstand wird an einer kleinen Kolonne destilliert. Ausbeute : 7,9g (81,4% d. Th.) an Verbindung 2.

### Beispiel 2

Herstellung von 5-tert-butyl-7a-methyl-3,3a,4,7-tetrahydro-2H-benzofuran (Verbindung 2) über die Syntheseroute II

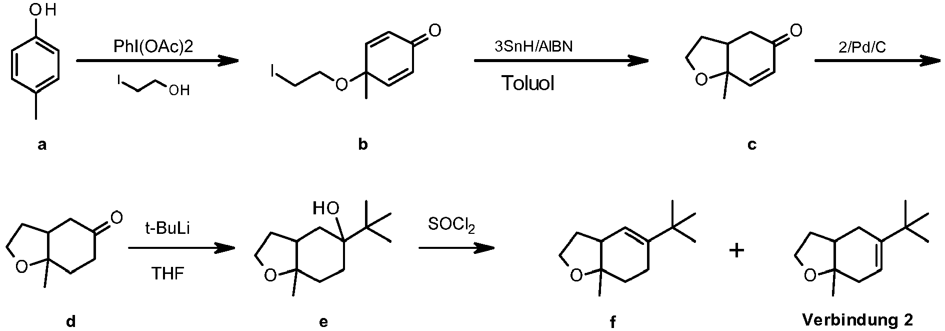

### Synthese von Zwischenverbindung b

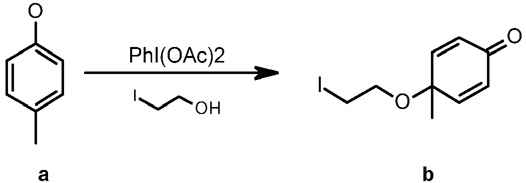

262 g (814mmol) Phl(OAc)2 und 223 g (1,61mol) K2C03 werden in einer Rührapparatur mit Septum Thermometer und Kühler unter N2 vorgelegt. Unter Rühren und Kühlen werden 800 ml 2-Jodethanol bei 0°C zugegeben. Anschließend werden nach 1h 80 g (740mmol) p-Kresol in 400ml 2-Jodethanol bei 0°C innerhalb von 1h zu getropft. Nach 12 h rühren bei Raumtemperatur wird mit 800ml gesättigter NaHC03-Lösung vorsichtig versetzt. Die untere Phase wird mit 1 N Na2S203 (800 mL) und gesättigter NaHC03 (800 mL) gewaschen. Überschüssiges 2-Jodethanol an einer kleinen Kolonne ab destilliert und über Kieselgel-Chromatographie gereinigt. Ausbeute : 63 g (30,6% d. Th.) an Verbindung b.

### Synthese von Zwischenverbindung c

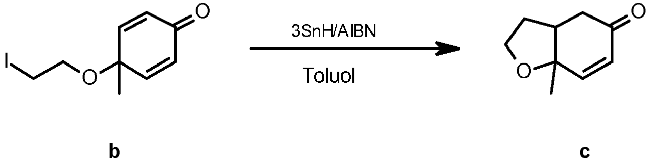

Zu einer Mischung aus 30 g (216mmol) Verbindung b in 500ml Toluol werden bei Raumtemperatur unter Kühlen 7,09 g (43,2mmol) AIBN und 75.4 g Bu3SnH (259 mmol) in 500mlToluol gegeben. Die Reaktionslösung wird 6 h unter Rückfluss gerührt. Toluol wird an einer kleinen Kolonne ab destilliert und über Kieselgel-Chromatographie gereinigt. Ausbeute: 19,6 g (59,7% d. Th.) an Verbindung c.

### Synthese von Zwischenverbindung d

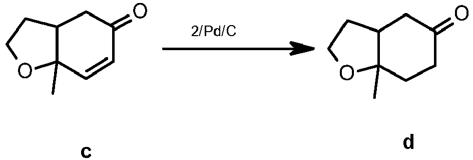

36.0 g, (237 mmol) Verbindung c in 250ml Essigester werden unter Zusatz von 500mg Pd/C 5%ig bei 40 °C und 5 bar 4 h hydriert. Nach abfiltrieren des Katalysators und ab destillieren des Essigesters erhalt man 31,0 g Verbindung d (wird direkt in der nächsten Stufe eingesetzt).

### Synthese von Verbindung 2

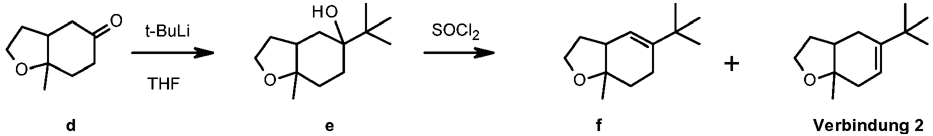

45 g (292 mmol) Verbindung d in 250ml THF abs. werden unter Rühren und Kühlen innerhalb von 1 h bei -60°C mit 225ml (292mmol) tert.-Butyllithium in Heptan versetzt. Die Reaktionslösung wird anschließend 12 h Raumtemperatur gerührt. Die Reaktionslösung wird unter Rühren und Kühlen innerhalb von 1 h mit 130ml SOCI2 versetzt und 4 h bei Raumtemperatur nachgerührt. Die Reaktionslösung wird eingeengt und über Kieselgel-Chromatographie gereinigt. Ausbeute: 3,08g Verbindung f und 0,52g Verbindung 2.
Verbindung f 1H NMR: (400 MHz, CDCl3)
δ 5.46 (tt, J = 4.4, 1.0 Hz, 1H), 3.82 (td, J = 8.2, 4.1 Hz, 1H), 3.72 (td, J = 8.2, 6.6 Hz, 1H), 2.25 - 2.20 (m, 1H), 2.20 - 2.13 (m, 1H), 2.06 (dd, J = 6.1, 1.9 Hz, 1H), 2.04 - 2.01 (m, 1H), 2.00 (dddd, J = 5.5, 4.5, 2.8, 1.5 Hz, 1H), 1.98 - 1.93 (m, 0H), 1.71 - 1.55 (m, 2H), 1.20 (s, 3H), 1.02 (s, 9H).
Verbindung 2 1H NMR: (400 MHz, CDCl3)
δ 5.31 (ddd, J = 4.0, 1.7, 1.0 Hz, 1H), 3.85 (td, J = 7.8, 4.8 Hz, 1H), 3.71
(td, J = 7.8, 6.7 Hz, 1H), 2.42 (ddq, J = 9.1, 3.8, 2.0 Hz, 1H), 2.24 (dq, J = 12.0, 7.6 Hz, 1H), 2.15 - 2.01 (m, 1H), 1.94 (dddt, J = 16.8, 6.2, 4.9, 1.2 Hz, 1H), 1.78 (ddd, J = 13.1, 6.1, 4.9 Hz, 1H), 1.68 - 1.56 (m, 1H), 1.51 (ddd, J = 13.4, 8.9, 4.9 Hz, 1H), 1.21 (s, 3H), 1.02 (s, 9H).

### Beispiele 3 und 4: Parfümöle

| | **Bsp.3** | **Bsp. 4** |
|---|---|---|
| AMBERWOOD® F | 7,000 | 7,000 |
| AMBRETTOLIDE | 6,000 | 6,000 |
| Verbindung 1, 2, 6, od. 8 | 0,000 | 34,000 |
| BHT IONOL | 3,000 | 3,000 |
| CASSIS BASE 345 BB | 10,000 | 10,000 |
| CITRONELLOL 950 | 10,000 | 10,000 |
| CITRONELLYL ACETATE EXTRA | 1,000 | 1,000 |
| DAMASCENONE 10% DPG | 5,000 | 5,000 |
| DAMASCONE ALPHA 10% DPG | 1,000 | 1,000 |
| DIPROPYLENE GLYCOL | 209,000 | 175,000 |
| ETHYLENE BRASSYLATE | 325,000 | 325,000 |
| GERANIOL SUPER | 20,000 | 20,000 |
| GERANYL ACETATE PURE | 1,000 | 1,000 |
| HEDIONE | 120,000 | 120,000 |
| HEDIONE HC/30 | 30,000 | 30,000 |
| HELIONAL | 4,000 | 4,000 |
| HEXENYL ACETATE CIS TRANS-3 10% DPG | 3,000 | 3,000 |
| HEXYL CINNAMIC ALDEHYDE ALPHA | 20,000 | 20,000 |
| HYDROXY CITRONELLAL | 15,000 | 15,000 |
| INDOLE FF 10% DPG | 1,000 | 1,000 |
| IONONE BETA | 8,000 | 8,000 |
| ISO E SUPER | 100,000 | 100,000 |
| LEMON OIL WINTER BERGAPTEN FREE | 10,000 | 10,000 |
| LINALOOL | 5,000 | 5,000 |
| LINALYL ACETATE | 25,000 | 25,000 |
| MAGNOLAN | 20,000 | 20,000 |
| MANDARIN OIL DIST. DECOL. | 5,000 | 5,000 |
| METHYL IONONE GAMMA PURE / IFF | 1,000 | 1,000 |
| METHYL OCTIN CARBONATE 1% DPG | 5,000 | 5,000 |
| NEROL 900 | 2,000 | 2,000 |
| PHENYLETHYL ALCOHOL | 20,000 | 20,000 |
| ROSESSENCE 193E | 3,000 | 3,000 |
| VERTOCITRAL 10% DPG | 5,000 | 5,000 |

mit Zusatz von Verbindung 1, 2, 6, od. 8 (siehe Liste) riecht die Mischung neben einer intensiveren Ambra- sowie Holznote harmonischer, blumiger und natürlicher.

### Beispiele 5 und 6: Parfümöle

| | Bsp. 5 | Bsp. 6 |
|---|---|---|
| AMBRETTOLIDE | 15,000 | 15,000 |
| Verbindung 2, 3, 4, 5, oder 7 | 0,000 | 40,000 |
| BASIL OIL COMORES TYPE E 10% DPG | 2,000 | 2,000 |
| BERGAMOT OIL | 40,000 | 40,000 |
| BOURGEONAL 10% DPG | 5,000 | 5,000 |
| CARDAMOM OIL | 2,000 | 2,000 |
| CASHMERAN | 15,000 | 15,000 |
| CINNAMON BARK OIL CEYLON 10% DPG | 5,000 | 5,000 |
| CLARY SAGE OIL | 2,000 | 2,000 |
| CORIANDER OIL | 3,000 | 3,000 |
| COUMARIN | 16,000 | 16,000 |
| DAMASCENONE | 2,000 | 2,000 |
| DIHYDRO MYRCENOL | 2,000 | 2,000 |
| DIPROPYLENE GLYCOL | 52,000 | 12,000 |
| FIR BALSAM ABS. 10% DPG | 5,000 | 5,000 |
| FLOROSA | 5,000 | 5,000 |
| GALAXOLIDE 50% IN DPG | 150,000 | 150,000 |
| GERANIOL SUPER | 3,000 | 3,000 |
| GERANYL ACETATE 60 | 2,000 | 2,000 |
| GLOBALIDE® | 210,000 | 210,000 |
| GUAIAC WOOD OIL | 5,000 | 5,000 |
| HEDIONE | 30,000 | 30,000 |
| HEDIONE HC/30 | 60,000 | 60,000 |
| HEXENOL CIS-3 10% DPG | 5,000 | 5,000 |
| ISO E SUPER | 130,000 | 130,000 |
| ISOBUTYL QUINOLINE DL 100% 1% DPG | 5,000 | 5,000 |
| JAVANOL | 3,000 | 3,000 |
| LAVANDINOIL ABRIALIS NAT. | 3,000 | 3,000 |
| LEMON OIL WINTER ITALIE | 15,000 | 15,000 |
| LINALOOL | 15,000 | 15,000 |
| LINALYL ACETATE | 25,000 | 25,000 |
| METHYL IONONE GAMMA PURE / IFF | 3,000 | 3,000 |
| MUSCENONE | 4,000 | 4,000 |
| PATCHOULI OIL DECOL. | 5,000 | 5,000 |
| SANDALWOOD OIL EAST IND. 10% DPG | 5,000 | 5,000 |
| SANDRANOL® | 10,000 | 10,000 |
| TONALIDE | 130,000 | 130,000 |
| VANILLIN | 3,000 | 3,000 |
| VERTOCITRAL 10% DPG | 5,000 | 5,000 |
| VETIVER OIL HAITI | 3,000 | 3,000 |

mit Zusatz von Verbindung 2, 3, 4, 5, oder 7 hat die Mischung neben einer intensiveren Ambra- sowie Holznote eine größere Strahlung und wirkt runder und harmonischer.

## Patentansprüche

1. Verbindung der Formel I: 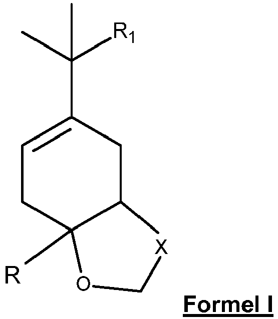
mit R = -methyl oder -ethyl,
R₁₌-H, -methyl, ethyl, O-methyl oder O-ethyl
X= -CH2- oder -CH2-CH2-.

2. Verbindung der Formel I nach Anspruch 1 mit
R = -methyl oder ethyl
R₁= -H, -methyl oder O-methyl und
X= -CH2- oder -CH2-CH2-.

3. Verbindung der Formel I nach Anspruch 1 ausgewählt aus der Gruppe enthaltend:

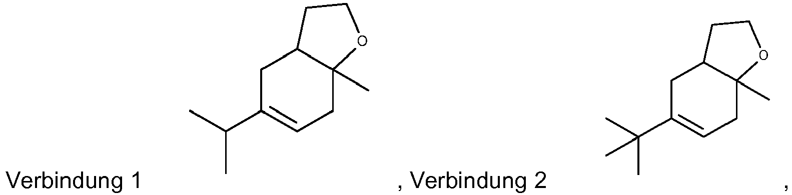

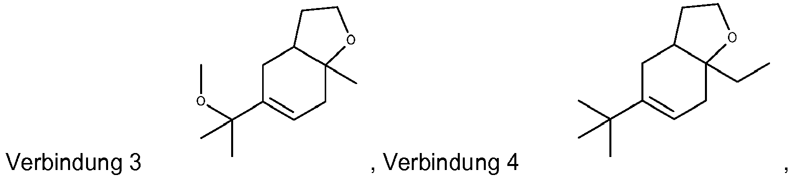

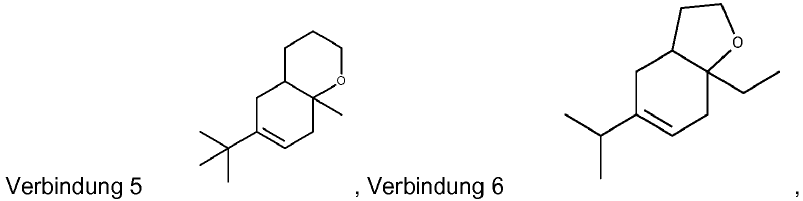

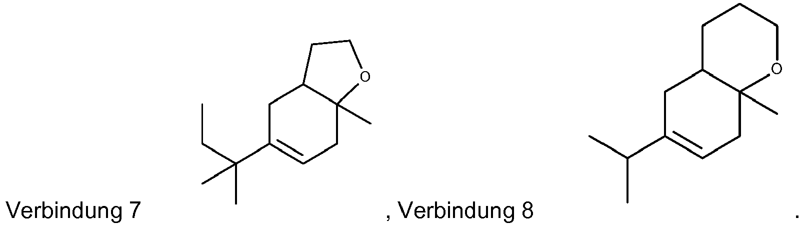

4. Mischung enthaltend
a) eine oder mehrere Verbindungen der Formel I nach einem der Ansprüche 1 bis 3
b) ein oder mehrere Lösungsmittel
c) sowie optional einen oder mehrere Duftstoffe.

5. Mischung enthaltend
a) eine oder mehrere Verbindungen der Formel I nach einem der Ansprüche 1 bis 3 und
c) einen oder mehrere Duftstoffe.

6. Konsumgüter enthaltend eine oder mehrere Verbindungen der Formel I nach einem der Ansprüche 1 bis 3.

7. Konsumgüter enthaltend Mischungen nach Anspruch 4 oder 5.

8. Verwendung einer oder mehrerer Verbindungen nach einem der Ansprüche 1 bis 3 als Duftstoffe.

9. Verwendung nach Anspruch 8 zur Erzeugung, Vermittlung, Modifizierung oder Verstärkung eines ambrartigen und / oder holzigen Geruchs.

10. Verfahren zur Herstellung von Verbindungen der Formel I nach Anspruch 1 umfassend
die Cyclisierung einer Verbindung der Formel II 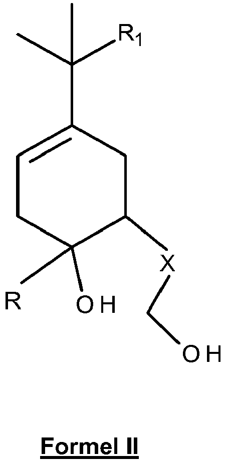
oder die Dehydratisierung von Verbindungen der Formel III 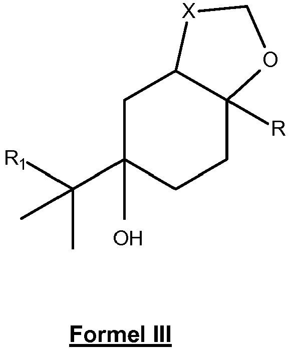
mit R = -methyl oder ethyl,
R₁ = -H, -methyl, ethyl, O-methyl, oder O-ethyl und
X= -CH2- oder -CH2-CH2-.

11. Verfahren nach Anspruch 10 in Gegenwart von Sulfonsäure oder Sulfonsäurederivaten.

12. Verfahren nach Anspruch 10 oder/und 11 in Gegenwart von Toluol.

13. Verfahren nach Anspruch 10, 11 oder/und 12, wobei die Temperatur zwischen 30 und 60°C beträgt.

14. Verfahren zur Parfümierung von Konsumgütern umfassend das Hinzufügen einer Verbindung der Formel I nach einem der Ansprüche 1 bis 3 oder einer Mischung nach einem der Ansprüche 4 oder 5 zu Konsumgütern.

## Claims

1. Compound of formula I: 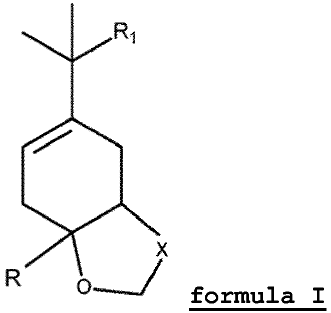
where R = -methyl or -ethyl,
R₁ = -H, -methyl, ethyl, O-methyl or O-ethyl,
X = -CH2- or -CH2-CH2-.

2. Compound of formula I according to claim 1, where
R = -methyl or ethyl,
R₁ = -H, -methyl or O-methyl, and
X = -CH2- or -CH2-CH2-.

3. Compound of formula I according to claim 1, selected from the group containing:

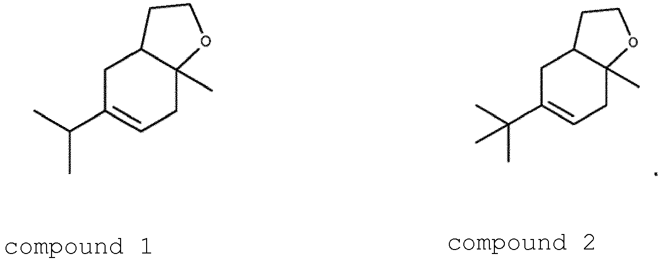

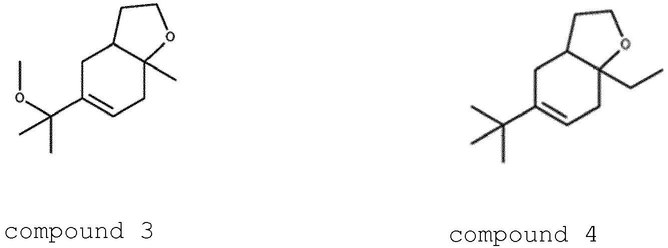

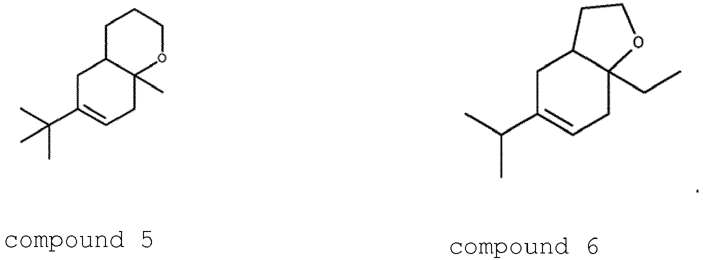

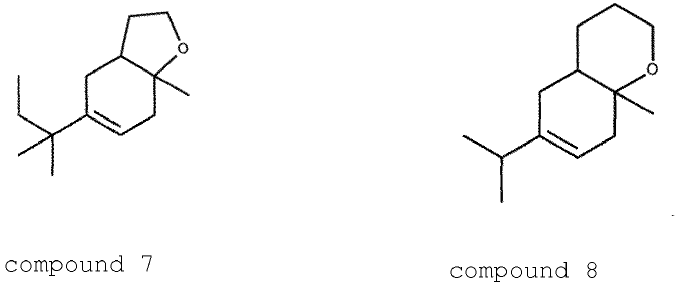

4. Mixture containing
a) one or more compounds of formula I according to one of claims 1 to 3,
b) one or more solvents,
c) and optionally one or more fragrant substances.

5. Mixture containing
a) one or more compounds of formula I according to one of claims 1 to 3, and
c) one or more fragrant substances.

6. Consumer goods containing one or more compounds of formula I according to one of claims 1 to 3.

7. Consumer goods containing mixtures according to claim 4 or 5.

8. Use of one or more compounds according to one of claims 1 to 3 as fragrant substances.

9. Use according to claim 8 for creating, imparting, modifying or enhancing an ambery and/or woody scent.

10. Method for producing compounds of formula I according to claim 1, which comprises
cyclizing a compound of formula II 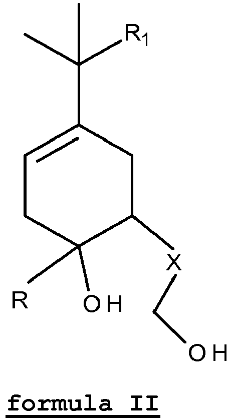
or dehydrating compounds of formula III 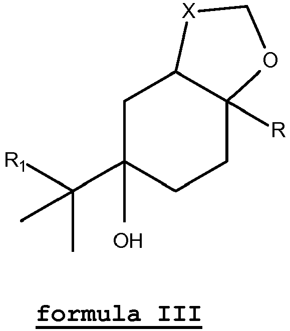
where R = -methyl or -ethyl,
R₁ = -H, -methyl, ethyl, O-methyl or O-ethyl, and
X = -CH2- or -CH2-CH2-.

11. Method according to claim 10 in the presence of sulphonic acid or sulphonic acid derivatives.

12. Method according to claim 10 or/and 11 in the presence of toluene.

13. Method according to claim 10, 11 or/and 12, wherein the temperature is between 30 and 60°C.

14. Method for fragrancing consumer goods, which comprises adding a compound of formula I according to one of claims 1 to 3 or a mixture according to one of claims 4 or 5 to consumer goods.

## Revendications

1. Composé de formule I : 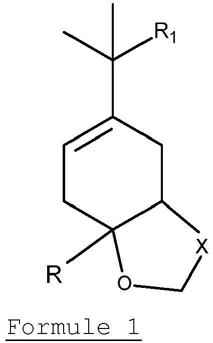
où R = un groupe méthyle ou éthyle,
R₁ = -H, un groupe méthyle, éthyle, O-méthyle ou O-éthyle,
X = -CH2- ou -CH2-CH2-.

2. Composé de formule I selon la revendication 1, où
R = groupe méthyle ou éthyle,
R1= -H, un groupe méthyle ou O-méthyle et
X = -CH2- ou -CH2-CH2-.

3. Composé de formule I selon la revendication 1, sélectionné dans le groupe comprenant :

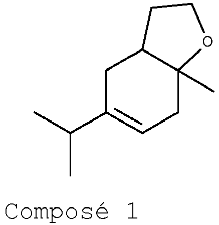

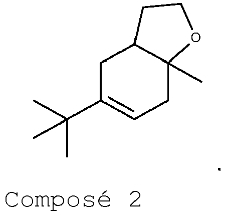

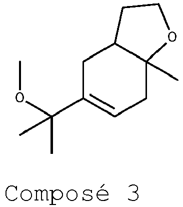

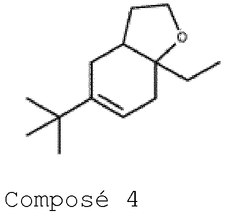

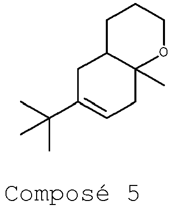

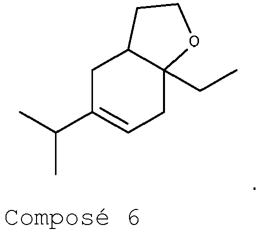

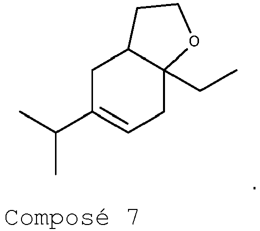

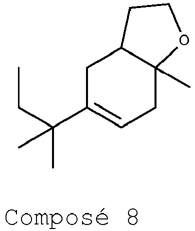

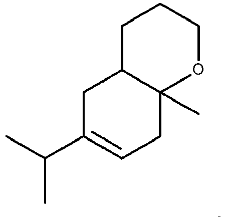

4. Mélange, comprenant
a) un ou plusieurs composés de formule I selon l'une des revendications 1 à 3,
b) un ou plusieurs solvants,
c) ainsi que, facultativement, une ou plusieurs fragrances.

5. Mélange, comprenant
a) un ou plusieurs composés de formule I selon l'une des revendications 1 à 3, et
c) une ou plusieurs fragrances.

6. Produits consommables, comprenant un ou plusieurs composés de formule I selon l'une des revendications 1 à 3.

7. Produits consommables, comprenant des mélanges selon la revendication 4 ou la revendication 5.

8. Utilisation comme fragrances d'un ou de plusieurs composés selon l'une des revendications 1 à 3.

9. Utilisation selon la revendication 8 pour la production, la diffusion, la modification ou le renforcement d'une senteur ambrée et / ou boisée.

10. Procédé de fabrication de composés de formule I selon la revendication 1, comprenant
la cyclisation d'un composé de formule II 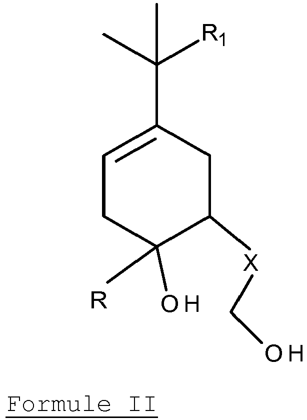 ou la déshydratation de composés de formule III 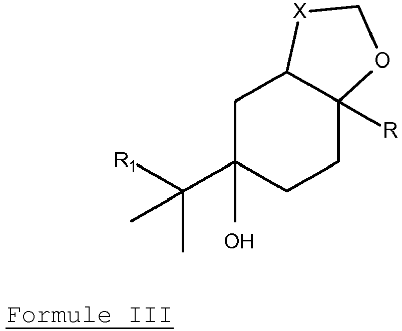
où R = un groupe méthyle ou éthyle,
R1 = -H, un groupe méthyle, éthyle, O-méthyle, ou O-éthyle et
X = -CH2- ou -CH2-CH2-.

11. Procédé selon la revendication 10, en présence d'acide sulfonique ou de dérivés d'acide sulfonique.

12. Procédé selon la revendication 10 et/ou la revendication 11, en présence de toluène.

13. Procédé selon la revendication 10, la revendication 11 et/ou la revendication 12, où la température est comprise entre 30 et 60°C.

14. Procédé pour parfumer des produits consommables, comprenant l'ajout d'un composé de formule I selon l'une des revendications 1 à 3 ou d'un mélange selon l'une des revendications 4 ou 5 à des produits consommables.
